# EUROPÄISCHE PATENTANMELDUNG

(11) **EP 0 995 421 A2**
(43) Veröffentlichungstag der Anmeldung: **26.04.2000**
(21) Anmeldenummer: 99121029.5
(22) Anmeldetag: 21.10.1999
(51) Int. Cl.: A61K 6/083, A61K 6/09, A61K 6/093

(54) **Lichtpolymerisierbares Einkomponenten-Dentalmaterial**

(30) Priorität: 23.10.1998 DE 19848886
(71) Anmelder: Heraeus Kulzer GmbH & Co.KG, 63450 Hanau (DE)
(72) Erfinder: Stange, Frank, 61250 Usingen (DE); Erdrich, Albert, Dr., 61231 Bad Nauheim (DE); Savic, Novica, 61273 Wehrheim (DE); Puchalska, Teresa, 61267 Neu-Anspach (DE)
(74) Vertreter: Kühn, Hans-Christian

(57) **Zusammenfassung**

Es wird u. a. ein lichtpolymerisierbares Einkomponenten-Dentalmaterial bereitgestellt, mit :
a) 80 - 10 Gewichts-% mindestens eines mehrfunktionellen Urethanmethacrylates und/oder mindestens eines mehrfunktionellen Urethanacrylates,
b) 10 - 30 Gewichts-% mindestens eines mehrfunktionellen Acrylatharzes,
c) 10 - 30 Gewichts-% mindestens eines Reaktiv-Verdünners,
d) 0 - 20 Gewichts-% Bis-GMA und/oder mindestens eines ethoxylierten Bisphenol-A-Dimethacrylates,
e) 0 - 10 Gewichts-% mindestens eines Füllstoffes,
f) 0 - 1 Gewichts-% mindestens eines Photoinitiatorsystems und
g) 0 - 1 Gewichts-% mindestens eines Farbpigments.

## Beschreibung

Die Erfindung betrifft ein lichtpolymerisierbares Einkomponenten-Dentalmaterial, ein Dental-Kit, ein Verfahren zur Herstellung einer Prothese, die Verwendung eines solchen Dentalmaterials, eine entsprechende Prothese, einen Fixierschlüssel, ein weiteres Dental-Kit, ein weiteres Verfahren zur Herstellung von Prothesen, die Verwendung eines bestimmten Materials als Fixierschlüssel und schließlich eine weitere Prothese.

Die auf dem Dentalmarkt hauptsächlich eingesetzten Systeme zur Restauration sind Zweikomponentensysteme auf Pulver-Flüssigkeitsbasis, die im Gießverfahren oder der Stopf-Preß-Technik verarbeitbar sind. Die Aushärtung erfolgt über eine Härtung im Wasserbad oder im Mikrowellengerät, wobei häufig die entstehenden Restaurationsgebilde, insbesondere Prothesen, zur Vermeidung von Siedeblasen mit Druck beaufschlagt werden.

Nachteilig hieran sind die Notwendigkeit des Anmischens, so daß bei zuviel angemischtem Material dieses verworfen werden muß. Des weiteren beinhalten diese Systeme MMA und Peroxide, die ein hohes biologisches Reizpotential besitzen. Darüber hinaus ist durch die Anquellung der Polymere und durch den Reaktionsstart bei Autopolymerisaten die Verarbeitungszeit in der Regel sehr begrenzt.

Weit weniger verbreitet sind die Einkomponentensysteme, die bisher hauptsächlich für den Markt der Totalprothetik eingesetzt werden. Diese Systeme beinhalten heißhärtende, teigartige Materialien, was zur Folge hat, daß eine Verarbeitung im Gießverfahren nicht möglich ist und - wie in der Totalprothetik üblich - eine Einbettung in Gips vorgenommen werden muß.

Weiterhin ist die Polymerisation sehr langsam bzw. die nachgeschaltete Abkühlungsphase überproportional lang. Der Kunststoffteig enthält in der Regel in nachteiliger Weise Peroxid als Initiator, der - wie schon oben erwähnt - ein hohes biologisches Reizpotential aufweist. Die Nachbearbeitung, besonders bei glasgefüllten Systemen ist sehr aufwendig, wobei darüber hinaus die Plaqueaffinität sehr hoch ist. Schließlich sind die werkstofftechnischen Eigenschaften in der Regel durch die schlechte Einbindung der Füllstoffe unbefriedigend.

Aus dem vorgenannten ergibt sich das Problem, mit Hilfe eines neuartigen Einkomponenten-Dentalmaterials und u. a. eines neuartigen Dental-Kits die oben genannten Nachteile zumindest teilweise zu beseitigen. Das sich ergebende Problem liegt insbesondere darin, ein Einkomponenten-Dentalmaterial und ein entsprechendes Kit bereitzustellen, bei denen die Herstellungszeiten im Vergleich zu üblichen Verfahren und Materialien deutlich kürzer sind, kein freies Methylmethacrylat und nahezu kein Peroxid verarbeitet wird, eine Anbindung an marktübliche PMMA-Materialien möglich sein soll und schließlich die Bearbeitung nach Polymerisation vom Aufwand her im Vergleich zu bisherigen Systemen ähnlich sein sollte.

Dieses Problem wird erfindungsgemäß durch ein Einkomponenten-Dentalmaterial nach Anspruch 1, ein Dental-Kit nach Anspruch 16, ein Verfahren nach Anspruch 17, eine Verwendung nach Anspruch 18, ein Prothese nach Anspruch 19, einen Fixierschlüssel nach Anspruch 24, ein Dental-Kit nach Anspruch 28, ein Verfahren nach Anspruch 29, eine Verwendung nach Anspruch 30 und durch eine Prothese nach Anspruch 31 gelöst.

Beim erfindungsgemäßen Einkomponenten-Dentalmaterial handelt es sich um ein solches, das mindestens ein mehrfunktionelles Urethanmethacrylat und/oder mindestens ein mehrfunktionelles Urethanacrylat, mindestens ein mehrfunktionelles Acrylatharz und mindestens einen Reaktiv-Verdünner aufweist. Unter dem Begriff Reaktiv-Verdünner sind im Erfindungskontext Verbindungen zu verstehen, die quasi als "Lösungs- und Verdünnungsmittel" in ihrer Monomerform wirken und somit die Viskosität des Einkomponenten-Dentalmaterials herabsetzen und darüber hinaus bei der Lichthärtung, also bei der Polymerisation mit Hilfe von Lichtwellenlängen, mit in die Polymermatrix einpolymerisiert werden.

Darüber hinaus kann das erfindungsgemäße Material Bis-GMA und/oder mindestens ein ethoxyliertes Bisphenol A-Dimethacrylat enthalten, das sich positiv auf Werkstoffparameter wie Schrumpf, Wasseraufnahme und mechanische Eigenschaften auswirkt. Bis-GMA ist die gängige Abkürzung für 2,2-Bis(4-(2-hydroxy-3-methacryloyloxypropoxy)phenyl)propan und weist folgende Strukturformel auf:

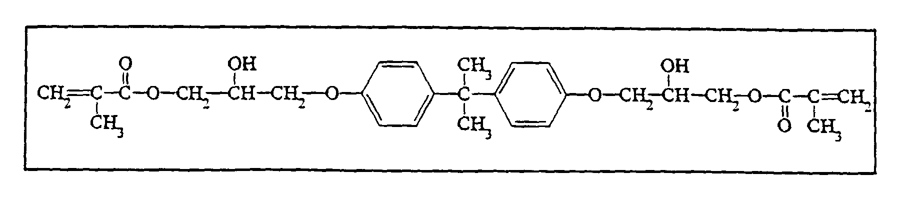

Schließlich können Füllstoffe, Photoinitiatorsysteme und Farbpigmente Bestandteile des Materials sein. Die Füllstoffe können hydrophobiert, beispielsweise silanisiert, sein, was die Benetzbarkeit aufgrund des geringeren polaren Charakters verbessert, was zu einer leichteren Einpolymerisierbarkeit in die Matrix führt. Als ein Beispiel von vielen sei Methacryloyloxypropyl-trimethoxy-silan genannt.

Das Photoinitiatorsystem kann beispielsweise aus einzelnen Photoinitiatoren oder aber aus Systemen bestehen, die Photoinitiatoren und Co-Initiatoren beinhalten.

Die Farbpigmente können organischer Natur, beispielsweise auf Perylen-Basis, oder anorganischer Natur, beispielsweise auf Spinell-Basis, sein.

Die in den Ansprüchen 2 bis 15 aufgeführten Ausgestaltungen des Dentalmaterials sind vorteilhafte Ausgestaltungen, da sich diese in der Praxis bewährt haben.

Das Urethanmethacrylat ist das Produkt aus der Umsetzung eines Diisocyanates mit einem OH-funktionellen Methacrylat wie beispielsweise Hydroxyethylmethacrylat. Bei Verwendung eines Diisocyantes ergibt sich als Produkt ein Urethandimethacrylat; bei Einsatz eines OH-funktionellen Acrylates wie etwa Hydroxyethylacrylat ergibt sich analog zum Methacrylat ein difunktionelles Acrylat.

Vorteilhaft ist ein solches Urethanmethacrylat oder Urethanacrylat, insbesondere ein Urethandimethacrylat, unter anderem deshalb, weil es überlegene Werkstoffeigenschaften wie hohe Steifigkeit oder geringe Wasseraufnahme bietet.

Möglich ist auch der Einsatz eines aus der Kombination von Triisocyanaten oder höherwertigeren Isocyanaten mit OH-funktionellen Acrylaten oder Methacrylaten hergestellten Monomers, wobei diese Urethanmethacrylate bzw. Urethanacrylate folglich eine Funktionalität von 3 oder höher aufweisen.

Weiterhin ist es von Vorteil, wenn das Urethanmethacrylat und/oder das Urethanacrylat bei T = +20 °C eine Viskosität von 10 ³ bis 5 x 10⁴ mPas, insbesondere von 1 x 10⁴ mPas, aufweist, da bei Einsatz im Einkomponenten-Dentalmaterial als Hauptmonomer bereits auch ohne Verdünnung eine für den erfindungsgemäßen Dentalkunststoff günstige Konsistenz gegeben ist.

Vorteilhafterweise weist das Urethanmethacrylat und/oder das Urethanacrylat eine Molmasse von 450 bis 500 g/mol auf, da der Volumenschrumpf des Monomers im Bereich normaler Prothesenwerkstoffe liegt. Beispielsweise liegt der Volumenschrumpf des Monomers bei einer Funktionalität von 2 (also ein Urethandimethacrylat oder ein Urethandiacrylat) unter 7 %.

Es ist von Vorteil, wenn das Urethanmethacrylat und/oder das Urethanacrylat aliphatisch ist, da solche Urethanmethacrylate und Urethanacrylate speziell bei Bestrahlung mit UV-haltigem Licht ein wesentlich geringeres Verfärbungsverhalten im Vergleich zu aromatischen Urethanmethacrylaten bzw. Urethanacrylaten aufweisen.

Bewährt hat sich das folgende Urethanmethacrylat folgender Strukturformel:

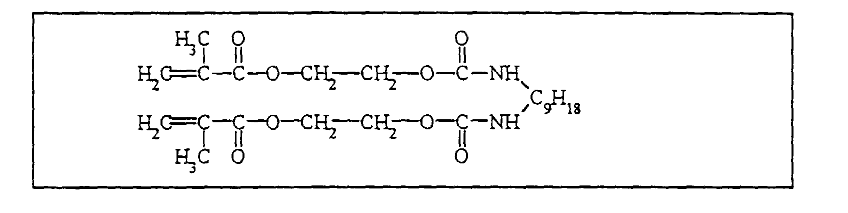

Vorteilhaft ist, daß das Acrylatharz ein Polyestertriurethanacrylat ist, da sich durch die Funktionalität von 3 trotz der hohen Molmasse noch eine im Verhältnis hohe Steifigkeit ergibt, die durch den E-Modul charakterisiert ist. Durch die langen Ketten zwischen den funktionellen Gruppen ergibt sich gleichzeitig eine hohe Flexibilität, was zu einer deutlichen Erhöhung der Schlagzähigkeit führt.

In vorteilhafter Weise weist das Acrylatharz bei T = + 60 °C eine Viskosität von 5.000 bis 15.000 mPas auf, da die hohe Viskosität dieses Monomers eine gute Abstimmung der Mischungsviskosität durch Variation mit Reaktiv-Verdünner und Hauptmonomer (mehrfunktionelles Urethanmethacrylat oder Urethanacrylat) gestattet.

Es hat sich besonders bewährt, wenn das Acrylatharz eine Molmasse von 1.000 bis 1.200 g/mol aufweist, da sich durch die hohe Molmasse ein günstiger Volumenschrumpf von unter 5 % bei der Polymerisation ergibt.

Weiterhin ist es von Vorteil, wenn das ethoxylierte Bisphenol-A-Dimethacrylat eine in Anspruch 11 definierte Strukturformel aufweist, wobei die Indices X und Y der Strukturformel im Bereich von 1 bis 4 liegen, da Bis-GMA als auch die entsprechenden ethoxylierten Bisphenol-A-Dimethacrylate sich durch niedrigen Volumenschrumpf und gute mechanische Eigenschaften auszeichnen, wobei letztere Verbindungen darüberhinaus eine sehr geringe Wasseraufnahme und Wasserlöslichkeit aufweisen.

In vorteilhafter Weise ermöglicht die Verwendung von Siliziumdioxid insbesondere die Verwendung von hochdispersen pyrogenen Kieselsäuren als Füllstoff eine präzise Einstellung des Fließverhaltens mit standfestem Verhalten in Ruhe und Fließen bei Belastungen wie Rühren oder Gießen (Thixotropie-Effekte). Durch Verwendung von silanisiertem Füllstoff wird neben der Erhöhung der Benetzbarkeit durch Verringerung des polaren Charakters sogar eine chemische Einbindung in die Matrix erzielt. Ein solcher Füllstoff wirkt als zusätzlicher Vernetzer und erhöht die Steifigkeit. Ein in die Matrix einpolymerisiertes Silan ist beispielsweise Methacryloyloxypropyl-trimethoxy-silan.

Ein weiteres wichtiges erfindungswesentliches Element ist ein Fixierschlüssel zur Herstellung von Prothesen, der mindestens ein transparentes Silikon oder Komposit enthält.

Im Erfindungskontext ist unter dem Begriff "Komposit" ein mit anorganischen, beispielsweise mit Glas, oder organischen Füllstoffen gefülltes und lichthärtendes Einkomponenten-Material zu verstehen.

Weiterhin ist im Erfindungskontext unter dem Begriff Fixierschlüssel ein zumindest teilweise ausgehärtetes Material zu verstehen, das zur Fixierung der Stellung der einzelnen Zähne zueinander dient.

Entscheidend bei der Erfindung ist die Tatsache, daß es sich um einen für die polymerisierende Aushärtung des erfindungsgemäßen Dentalmaterials im Lichtwellenlängenbreich transparenten Fixierschlüssel handelt.

Bei dem aus Silikon bestehenden Fixierschlüssel ist es von Vorteil, wenn das Silikon eine Shore-Härte von 50 bis 80 aufweist und ein vernetzendes Vinylpolysiloxan ist, da dieses eine hohe Transparenz, eine schnelle Aushärtung bei Raumtemperatur ohne technische Hilfsmittel und eine hohe Abformgenauigkeit der Aufstellung der Zähne im Wachs aufweist. Durch chemische Unverträglichkeiten von Silikon mit Methacrylaten und Acrylaten kommt es auch ohne Trennmittel zu keiner Anhaftung des Kunststoffes der Prothese an den Fixierschlüssel.

Weiterhin ist es vorteilhaft, wenn das Komposit ein mit Glas oder organischen Füllstoffen gefülltes, lichthärtendes Polymer ist, da dieses eine hohe mechanische Steifigkeit mit hoher Transparenz verbindet. In diesem Fall wird jedoch ein zusätzliches Trennmittel zur Isolierung von Komposit gegen den Kunststoff der Prothese benötigt.

Die beiden erfindungsgemäßen Dental-Kits enthalten mindestens ein erfindungsgemäßes Dentalmaterial oder mindestens ein transparentes, vernetzendes Silikon oder Komposit als Fixierschlüssel, wobei in vorteilhafter Weise ein solches Kit sowohl das Dentalmaterial als auch das Fixierschlüssel-Material enthält.

Darüber hinaus kann ein solches Kit einen lichthärtenden Haftvermittler zum Anbinden der Kunststoffzähne an die Prothese, ein Abdeckmaterial, beispielsweise ein Lack oder eine gelartige Paste, zur Vermeidung luftbedingter Dispersionsschichten sowie einen initiatorhaltigen Lack zur Verbesserung der Polymerisation an lichtunzugänglichen Stellen aufweisen.

Das Dentalmaterial als solches kann transparent als auch in gängigen Prothesenfarben eingefärbt und geadert sein. Unter Aderung ist die Zugabe von Kunststoff-Fasern (Länge bis 2 mm) zum bereits fertig eingefärbten Material zu verstehen, um einen möglichst natürlichen Gesamteindruck beim polymerisierten Material zu erreichen. Durch den Sauerstoff der Luft kann es zu einer Inhibierung der Polymerisationsreaktion unter Bildung von luftbedingten Dispersionsschichten kommen, wodurch an der Kunststoffoberfläche nach der Polymerisation eine dünne, unpolymerisierte Schicht zurück bleiben würde, was jedoch durch das obige Abdeckmaterial vermieden werden kann.

Das Dentalmaterial kann in Kartuschen, Spritzen oder Tuben dargereicht werden. Es weist ein bei der Verarbeitung thixotropes Verhalten auf, d. h. in diesem Fall kurzzeitiges Fließen unter Druck oder Bearbeitung, um anschließend standfest zu werden. Jedoch ist auch ein schichtweises Anpolymerisieren insbesondere über Handlichtgeräte denkbar.

Durch die Verwendung von ausschließlich mehrfachfunktionellen, insbesondere höhermolekularen, Monomeren, wird der Schrumpf gering gehalten. Dies ermöglicht es, ohne größere Füllstoffmengen auszukommen und vom Schrumpf her trotzdem im Bereich normaler Zwei-Komponenten-Gießkunststoffe zu bleiben (ca. 7 %). Der Verzicht auf größere Mengen Füllstoff umgeht das Problem der Anbindung von der Monomermatrix an die Füllstoffe. Die Bearbeitbarkeit (Beschleifen, Polieren) ist bei weitestgehendem Verzicht auf härtere Füllstoffe wie Glas etc. ähnlich der von herkömmlichen Werkstoffen. Die bei Verwendung mehrfachfunktioneller Monomere auftretende höhere Sprödigkeit wird durch Acrylatharz, insbesondere durch hochmolekulares Acrylatharz, größerer Flexibilität kompensiert. Die Verarbeitung erfolgt wie üblich über die Gießtechnik, die Aushärtung erfolgt jedoch durch Lichthärtung, beispielsweise durch Bestrahlen mittels Stroboskoplampen, was durch die Verwendung transparenter Fixierschlüssel erstmals möglich ist. Durch das Vermeiden des Anmischvorganges werden darüber hinaus eine immer gleichbleibende Verarbeitungskonsistenz und konstante Werkstoffeigenschaften des Materials sichergestellt.

Als über die Erstellung von Prothesen darüber hinausgehende Einsatzgebiete seien beispielsweise die Unterfütterung bei totalen Prothesen, die Reparatur voll- und teilprothetischer Arbeiten und die Anwendung im kieferorthopädischen Bereich erwähnt. Ferner ist mit transparentem Einbettmaterial eine Erweiterung auf den Bereich der Totalprothetik ohne weiteres möglich.

Die Dentalmaterial-Eigenschaften erfüllen die ISO 1567. Bisherige Muster ergaben einen E-Modul von ca. 2200 MPa und eine Biegefestigkeit von ca. 85 MPa, wobei die Schlagzähigkeit (DIN 53435) größer als 6 kJ/m² war.

Die erfindungsgemäßen Verfahren, Prothesen und Verwendungen weisen ebenfalls die oben angegebenen überraschenden und vorteilhaften Eigenschaften auf.

Das nachfolgende Beispiel dient zur Erläuterung der Erfindung.

Als erste Maßnahme bei der Versorgung eines Zahnlosen mit totalen Prothesen wird eine funktionelle Situationsabformung der Kiefer vorgenommen. Als Abformmaterialien werden dafür heute im allgemeinen Alginate verwendet, die - in Pulverform geliefert - mit Wasser zu einem Brei angemischt werden. Durch Zusatz weiterer Stoffe bildet sich beim Anmischen ein schwerlösliches, vernetztes Gel. Das teilweise vernetzte Gel wird an die jeweilige Stelle im Zahnbereich eines Patienten aufgebracht und so eine Abformung hergestellt. Analog zum Alginat können als Abformmaterial auch Materialien auf Silikonbasis verwendet werden.

Durch Ausgießen der Abformung, die eine Negativform darstellt, gewinnt man wieder ein Positiv, das Arbeitsmodell. Als Modellwerkstoff wird fast ausschließlich Gips verwendet.

### 1. Vorarbeiten

Auf das Arbeitsmodell aus Gips wird eine Aufstellung der Zähne in Wachs vorgenommen. Hierbei dient das Wachs als Platzhalter für den späteren Kunststoff, das Dentalmaterial. An diese Wachsaufstellung wird das zur Erstellung des Fixierschlüssels angemischte Silikon adaptiert. Das Zwei-Komponenten-Silikonmaterial mischt sich beim Auspressen selbst und härtet innerhalb von 3 bis 5 Minuten aus. Nach der Aushärtung des Silikons wird der Fixierschlüssel abgenommen, das Wachs entfernt und die Zähne im Silikon-Fixierschlüssel befestigt. Dieser wird anschließend wieder auf das Gipsmodell aufgesetzt. Später miteinzupolymerisierende Metallgerüste werden standardmäßig durch Sandstrahlen, Konditionieren und anschließendem Beschichten mit Opaker, d. h. einem opaken, farblich abgestimmten Metallabdecklack, vorbereitet.

### 2. Vorbereitung des Modells

Auf das feuchte Gipsmodell wird eine Alginatisolierung mit einem Pinsel in zwei dünnen Schichten aufgebracht und anschließend an der Luft getrocknet. Die Alginatisolierung trennt das im Gips enthaltene Wasser vom Kunststoff und dichtet darüber hinaus die Poren des Gipses ab. Auf diese Art und Weise werden sogenannte Weißverfärbungen, die durch die Einlagerung von Wasser im auspolymerisierten Kunststoff entstehen, wirksam unterbunden. Wegen späterer Abschattungen an kaum lichtzugängliche Stellen ist der Auftrag einer initiatorhaltigen Schicht zur Unterstützung der späteren Lichtpolymerisation vorteilhaft.

### 3. Konditionierung der Zähne/Metallgerüste

Die Basalflächen der Zähne werden mit einem Diamantschleifer angerauht und anschließend mit einem Haftvermittler, beispielsweise bestehend aus einem lichtinitiiertem, niedermolekularen Methacrylat, konditioniert. Im Gegensatz zu normalen Pulver-Flüssigkeitssystemen ist dieser Haftvermittler lichthärtend, um eine optimale Verbindung bei der Polymerisation zu gewährleisten. Speziell die bei der späteren Polymerisation schlecht bestrahlten Rückseiten der Metallgerüste werden zur Unterstützung der Aushärtung mit einer initiatorhaltigen Monomerschicht, beispielsweise bestehend aus höher initiiertem oder leicht peroxidhaltigem Basis-Dentalmaterial, bestrichen.

### 4. Verarbeitung des Gießkunststoffes

Der in einer Kartusche oder Tube verarbeitungsfertige Gießkunststoff, also das Dentalmaterial als solches, wird direkt in den Hohlraum zwischen Gips und Silikonvorwall (Fixierschlüssel) gegossen. Im Gegensatz zu marktüblichen Systemen kann das Anmischen, bei pastenartigen Materialien die Einbettung, vermieden werden. Bei extrem dicken Stücken oder aber bei opaken Farben kann es notwendig sein, den Kunststoff schichtweise zu polymerisieren. Der Kunststoff ist zwar nach dem Eingießen durch Thixotropieeffekte relativ standfest, kann jedoch zur Fixierung zusätzlich oberflächlich mit einem Handlichtgerät anpolymerisiert werden.

Anschließend erfolgt die Polymerisation mittels Blitzlichtgerät, wobei nach der ersten Polymerisation der Silikonvorwall entfernt wird, um anschließend nochmals zu belichten. Vor der zweiten, lediglich optionalen Belichtung werden freiliegende Kunststoffoberflächen zur Vermeidung der Inhibitionsschicht mit Antidispersionsschicht-Gel bestrichen. Dieses kann beispielsweise eine Glycerin-/Wasser-Basis enthalten.

### 5. Nachbearbeitung

Die Nachbearbeitung erfolgt in der Regel durch Beschleifen und Polieren.

## Patentansprüche

1. Lichtpolymerisierbares Einkomponenten-Dentalmaterial, mit:
a) 80 - 10 Gewichts-% mindestens eines mehrfunktionellen Urethanmethacrylates und/oder mindestens eines mehrfunktionellen Urethanacrylates,
b) 10 - 30 Gewichts-% mindestens eines mehrfunktionellen Acrylatharzes,
c) 10 - 30 Gewichts-% mindestens eines Reaktiv-Verdünners,
d) 0 - 20 Gewichts-% Bis-GMA und/oder mindestens eines ethoxylierten Bisphenol-A-Dimethacrylates,
e) 0 - 10 Gewichts-% mindestens eines Füllstoffes,
f) 0 - 1 Gewichts-% mindestens eines Photoinitiatorsystems und
g) 0 - 1 Gewichts-% mindestens eines Farbpigments.

2. Dentalmaterial nach Anspruch 1, dadurch gekennzeichnet, daß das Urethanmethacrylat ein Urethandimethacrylat oder ein Urethantrimethacrylat und das Urethanacrylat ein Urethandiacrylat oder ein Urethantriacrylat ist.

3. Dentalmaterial nach einem der vorhergehenden Ansprüche, dadurch gekennzeichnet, daß das Urethanmethacrylat und/oder Urethanacrylat bei T = + 20 °C eine Viskosität von 10³ bis 5 x 10⁴ mPas aufweist.

4. Dentalmaterial nach einem der vorhergehenden Ansprüche, dadurch gekennzeichnet, daß das Urethanmethacrylat und/oder Urethanacrylat eine Molmasse von 450 bis 500 g/mol aufweist.

5. Dentalmaterial nach einem der vorhergehenden Ansprüche, dadurch gekennzeichnet, daß das Urethanmethacrylat und/oder Urethanacrylat aliphatisch ist.

6. Dentalmaterial nach einem der vorhergehenden Ansprüche, dadurch gekennzeichnet, daß das Urethanmethacrylat folgende Strukturformel aufweist:

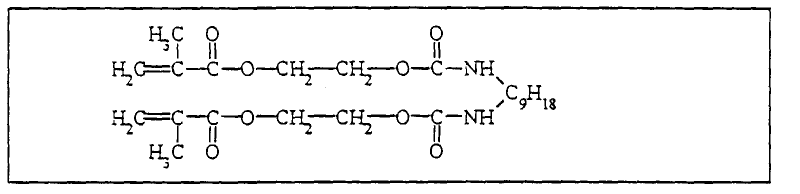

7. Dentalmaterial nach einem der vorhergehenden Ansprüche, dadurch gekennzeichnet, daß das Acrylatharz ein Polyestertriurethanacrylat ist.

8. Dentalmaterial nach einem der vorhergehenden Ansprüche, dadurch gekennzeichnet, daß das Acrylatharz bei T = + 60 °C eine Viskosität von 5000 bis 15000 mPas aufweist.

9. Dentalmaterial nach einem der vorhergehenden Ansprüche, dadurch gekennzeichnet, daß das Acrylatharz eine Molmasse von 1000 bis 1200 g/mol aufweist.

10. Dentalmaterial nach einem der vorhergehenden Ansprüche, dadurch gekennzeichnet, daß der Reaktiv-Verdünner folgende Strukturformel aufweist:
TRIM: Trimethylolpropantrimethacrylat 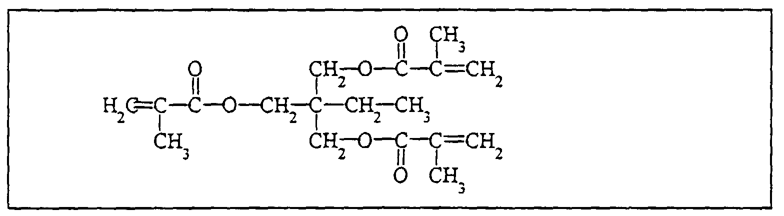 oder
TEDMA: Triethylenglykoldimethacrylat 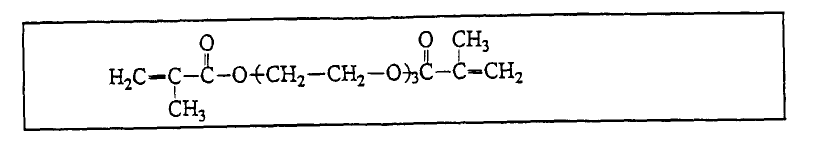 oder
Pentaerythritoltetraacrylat: mittlere n =2-3 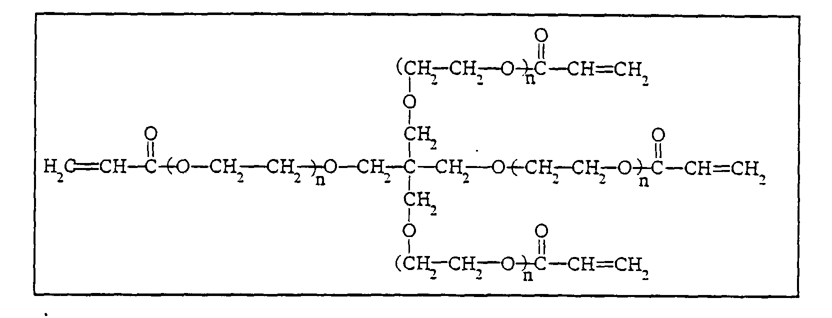 oder
DDMA: Dodecandioldimethacrylat 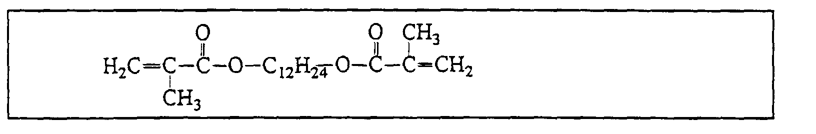 oder
Isobornylmethacrylat:

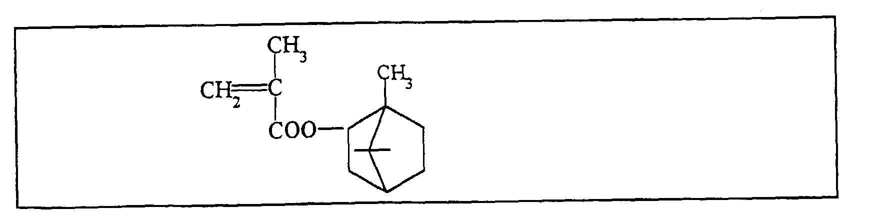

11. Dentalmaterial nach einem der vorhergehenden Ansprüche, dadurch gekennzeichnet, daß das ethoxylierte Bisphenol-A-Dimethacrylat folgende Strukturformel aufweist, wobei die Indices X und Y der Strukturfomel im Bereich von 1 bis 4 liegen:

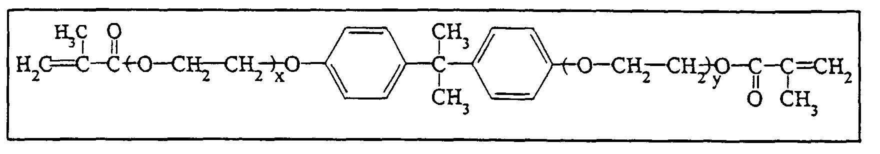

12. Dentalmaterial nach einem der vorhergehenden Ansprüche, dadurch gekennzeichnet, daß der Füllstoff Siliziumdioxid aufweist.

13. Dentalmaterial nach Anspruch 12, dadurch gekennzeichnet, daß der Füllstoff hochdisperse pyrogene Kieselsäuren enthält.

14. Dentalmaterial nach einem der vorhergehenden Ansprüche, dadurch gekennzeichnet, daß das Photoinitiatorsystem
2,4,6 -Trimethylbenzoyldiphenylphosphinoxid TMDPO 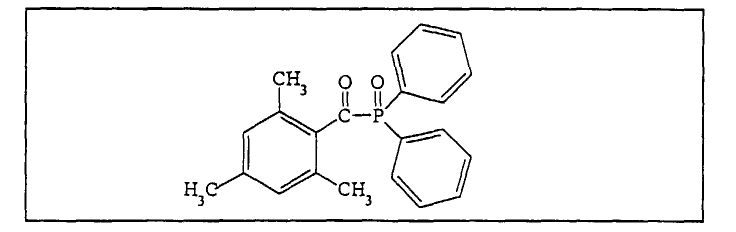 und/oder
2-Hydroxy-2-methyl-1-phenyl-propan-1-on 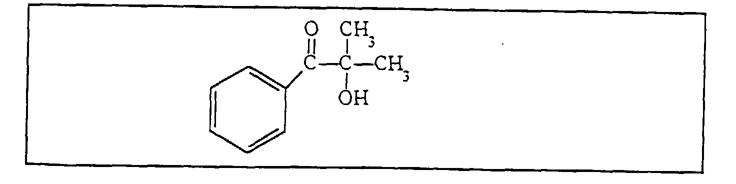 und/oder
2,3-Bornandion 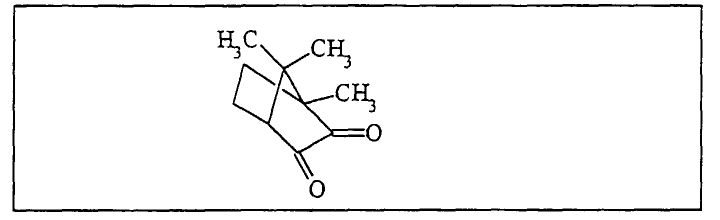 und/oder
2-n-Butoxyethyl-4-dimethylaminobenzoat 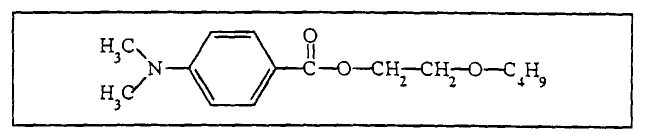 und/oder
PMA: Pentamethylanilin 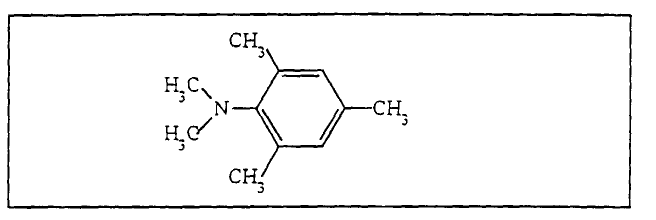 enthält.

15. Dentalmaterial nach einem der vorhergehenden Ansprüche, dadurch gekennzeichnet, daß das Farbpigment ein organisches Perylen-, ein Titandioxid- oder ein Kobaltzinkaluminatblauspinell-Pigment ist.

16. Dental-Kit, enthaltend mindestens ein Dentalmaterial nach einem der Ansprüche 1 bis 15.

17. Verfahren zur Herstellung einer Prothese, dadurch gekennzeichnet, daß ein Dentalmaterial nach einem der Ansprüche 1 bis 15 verwendet wird.

18. Verwendung eines Dentalmaterials nach einem der Ansprüche 1 bis 15 zur Herstellung einer Total- oder Teilprothese.

19. Prothese, dadurch gekennzeichnet, daß diese mindestens ein Dentalmaterial nach einem der Ansprüche 1 bis 15 enthält.

20. Dental-Kit nach Anspruch 16, enthaltend mindestens ein transparentes, vernetzendes Silikon oder Komposit als Fixierschlüssel.

21. Dental-Kit nach Anspruch 20, dadurch gekennzeichnet, daß das Silikon eine Shore-Härte von 50 bis 80 aufweist.

22. Dental-Kit nach einem der Ansprüche 20 bis 21, dadurch gekennzeichnet, daß das Silikon ein vernetzendes Vinylpolysiloxan ist.

23. Dental-Kit nach einem der Ansprüche 20 bis 22, dadurch gekennzeichnet, daß das Komposit ein mit Glas oder organischen Füllstoffen gefülltes, lichthärtendes Polymer ist.

24. Fixierschlüssel, dadurch gekennzeichnet, daß dieser mindestens ein transparentes Silikon oder Komposit enthält.

25. Fixierschlüssel nach Anspruch 24, dadurch gekennzeichnet, daß das Silikon eine Shore-Härte von 50 bis 80 aufweist.

26. Fixierschlüssel nach einem der Ansprüche 24 bis 25, dadurch gekennzeichnet, daß das Silikon ein vernetzendes Vinylpolysiloxan ist.

27. Fixierschlüssel nach einem der Ansprüche 24 bis 26, dadurch gekennzeichnet, daß das Komposit ein mit Glas oder organischen Füllstoffen gefülltes, lichthärtendes Polymer ist.

28. Dental-Kit, enthaltend mindestens ein transparentes, vernetzendes Silikon oder Komposit als Fixierschlüssel nach einem der Ansprüche 24 bis 27.

29. Verfahren zur Herstellung von Prothesen, dadurch gekennzeichnet, daß mindestens ein transparentes, vernetzendes Silikon oder Komposit als Fixierschlüssel nach einem der Ansprüche 24 bis 27 verwendet wird.

30. Verwendung von mindestens einem transparenten, vernetzenden Silikon oder Komposit als Fixierschlüssel nach einem der Ansprüche 24 bis 27.

31. Prothese, dadurch gekennzeichnet, daß diese nach einem Verfahren nach Anspruch 29 hergestellt ist.
